# EUROPÄISCHE PATENTANMELDUNG

(11) **EP 1 428 520 A2**
(43) Veröffentlichungstag der Anmeldung: **16.06.2004**
(21) Anmeldenummer: 03027425.2
(22) Anmeldetag: 28.11.2003
(51) Int. Cl.: A61K 7/32

(54) **Lipase-Inhibitoren in Deodorantien und Antitranspirantien**

(30) Priorität: 10.12.2002 DE 10257736
(71) Anmelder: Henkel Kommanditgesellschaft auf Aktien, 40589 Düsseldorf (DE)
(72) Erfinder: Banowski, Bernhard, 40597 Düsseldorf (DE); Wadle, Armin, Dr., 40699 Erkrath (DE); Siegert, Petra, Dr., 42781 Haan (DE); Sättler, Andrea, Dr., 40255 Düsseldorf (DE)

(57) **Zusammenfassung**

Gegenstand der Erfindung ist die Verwendung von ausgewählten Lipase-inhibierenden Substanzen in einer kosmetischen Deodorant- oder Antitranspirant-Zusammensetzung zur Verringerung des durch Zersetzung von natürlichen Fetten der Haut und der Kopfhaut verursachten Körpergeruchs.

## Beschreibung

Gegenstand der Erfindung ist die Verwendung von ausgewählten Lipase-inhibierenden Substanzen in einer kosmetischen Deodorant- oder Antitranspirant-Zusammensetzung zur Verringerung des durch die Zersetzung von natürlichen Fetten der Haut und der Kopfhaut verursachten Körpergeruchs.

Apokriner Schweiß stellt eine komplexe Mischung dar, die unter anderem Sebum und andere Fette, Steroide, Cholesterin sowie ca. 10 % Eiweiße enthält.
Die Entwicklung der kurz- und mittelkettigen Fettsäuren, die wesentlich zum Körpergeruch beitragen, beginnt mit der Spaltung von Hautlipiden zu verzweigten langkettigen Fettsäuren. Die Spaltung der Hautlipide, die überwiegend als Glycerinester vorliegen, erfolgt im Wesentlichen durch Propionibacterium, Corynebacterium A und Staphylococcus-Spezies (A.G. James et al., Generation and Turnover of Volatile Fatty Acids by Axillary Bacteria, 22^{nd} IFSCC Congress, Edinburgh, 2002, Poster 108). A.G. James et al. offenbaren weiterhin, dass aus den langkettigen verzweigten Fettsäuren durch die hydrolytischen Enzyme von einem bestimmten Corynebacterium, das A.G. James et al. als Corynebacterium A bezeichnen, sowohl kurzkettige C₂ - C₅-Fettsäuren als auch mittelkettige C₆ - C₁₂-Fettsäuren gebildet werden, die hauptsächlich für den axillaren Körpergeruch verantwortlich sind. Prinzipiell sind alle bakteriellen Exoesterasen zu dieser Lipidspaltung fähig, besonders aber das Enzym Lipase.

Die erfindungsgemäß wirksamen Deodorant-Zusammensetzungen können an dieser Stelle eingreifen und die Tätigkeit der bakteriellen Exoesterasen hemmen. Damit unterscheiden sie sich von den rein bakteriostatischen oder bakteriziden Zusammensetzungen des Standes der Technik, die den Nachteil aufweisen können, die natürliche Mikroflora der Haut zu beeinträchtigen.

Ein bekannter desodorierender Wirkstoff mit Esterase-inhibierender Aktivität ist Triethylcitrat. Die Bekämpfung von durch flüchtige Fettsäuren verursachtem Körpergeruch durch die Hemmung von Lipase ist im Stand der Technik ebenfalls bekannt. Bisher ist allerdings nur eine begrenzte Anzahl Lipase-inhibierender Wirkstoffe offenbart, z. B. Aminomethylenmalonsäure-Derivate (DE 3018132 A1), Ethylenoxid-Propylenoxid-Copolymere (GB 2335596 A1 ) oder die Salze der Phytinsäure (EP 650 720 A1).

Aufgabe der vorliegenden Erfindung war es, weitere Lipase-inhibierende Wirkstoffe zu identifizieren, um eine größere Variabilität, Flexibilität und Hautverträglichkeit bei der Formulierung kosmetischer Deodorantien zu ermöglichen. Die Identifizierung bekannter kosmetischer Wirkstoffe als Lipase-Inhibitoren ermöglicht darüber hinaus, die Dosierung dieser Wirkstoffe herabzusetzen. Die enzyminhibierende Wirkung zeigt sich häufig bereits bei niedrigen Wirkstoffkonzentrationen, bei denen noch keine bakteriostatische oder bakterizide Wirkung gefunden wird.

Gegenstand der vorliegenden Erfindung ist die Verwendung von mindestens einer Lipase-inhibierenden Substanz, ausgewählt aus
einbasigen C₂-C₈-Carbonsäuren mit 1 - 7 Hydroxygruppen und deren physiologisch verträglichen Salzen,
aromatischen Carbonsäuren mit 6 - 24 Kohlenstoffatomen, 1 Carboxylgruppe, 1 - 2 Phenylresten, 1 - 6 Hydroxy- und/oder Cyanogruppen sowie deren physiologisch verträglichen Salzen,
C₂-C₁₁-Aminosäuren, deren N-C₂-C₂₂-Acylderivaten sowie deren physiologisch verträglichen Salzen,
den Tetraestern von Pentaerythrit mit C₂-C₄-Carbonsäuren, die einen substituierten Arylrest enthalten,
gewünschtenfalls ethoxylierten Mono- und Diestern von Glycerin und Polyglycerinen mit gewünschtenfalls hydroxylierten C₈-C₂₂-Fettsäuren,
den Ethern und Estern von gewünschtenfalls alkylierten Mono-, Oligo- und Polysacchariden,
Pflanzenextrakten, ausgewählt aus den Extrakten von Centella asiatica, Apfelkernen, den Früchten (Beeren) von Phyllanthus emblica, Meeresalgen, Henna, den Blättern des Olivenbaumes (Olea europaea), der Rinde des Afrikanischen Zwetschgenbaumes (Pygeum africanum, Prunus africana), Weidenröschen (Epilobium angustifolium, Willowherb), Papayasaft, Zitronen und Palmarosaöl (Cymbopogon Martini Oil),
Flavonoiden, Polyphenolen, Ubichinonen, 2-(2H-Benzotriazol-2-yl)-6-alkylphenolderivaten, α-Bisabolol, 2,2-Dimethyl-3-phenyl-1-propanol, Papain (Papaya-Petidase I, EC 3.4.22.2), Chymopapain (Papaya-Petidase II, EC 3.4.22.6), Bromelain (EC 3.4.22.32 und EC 3.4.22.33), Ficin (Ficain, EC 3.4.22.3) und Asclepain (EC 3.4.22.7), Phenylpropyldimethylsiloxysilicaten sowie Aluminiumchlorohydrat,

in einer kosmetischen Deodorant- oder Antitranspirant-Zusammensetzung zur Verringerung des durch die hydrolytische Zersetzung von natürlichen Fetten der Haut und der Kopfhaut verursachten Körpergeruchs.

In einer bevorzugten Ausführungsform sind die erfindungsgemäß verwendeten einbasigen C₂-C₈-Carbonsäuren mit 1 - 7 Hydroxygruppen ausgewählt aus Glycolsäure, Milchsäure, α-Hydroxybuttersäure, α-Hydroxyvaleriansäure, α-Hydroxycapronsäure, Gluconsäure, Galactonsäure, Mannonsäure, Fructonsäure, Arabinonsäure, Xylonsäure, Ribonsäure und Glucoheptonsäure sowie deren physiologisch verträglichen Salzen, insbesondere Zinklactat, Aluminiumlactat, Aluminiumgluconat, Zinkgluconat, Mangangluconat und Magnesiumglucoheptonat.

Erfindungsgemäß werden die einbasigen C₂-C₈-Carbonsäuren mit 1 - 7 Hydroxygruppen und ihre physiologisch verträglichen Salze in Mengen von 0,001 bis 10 Gew.-%, bevorzugt 0,005 bis 5 Gew.-% und besonders bevorzugt 0,01 bis 2 Gew.-%, jeweils bezogen auf die gesamte kosmetische Zusammensetzung, eingesetzt.

In einer weiteren bevorzugten Ausführungsform sind die erfindungsgemäß verwendeten aromatischen Carbonsäuren mit 6 - 24 Kohlenstoffatomen, 1 Carboxylgruppe, 1 - 2 Phenylresten, 1 - 6 Hydroxy- und/oder Cyanogruppen, deren Ester sowie deren physiologisch verträglichen Salze ausgewählt aus Mandelsäure, para-Hydroxymandelsäure, Rosmarinsäure, Ferulasäure, Kaffeesäure, Chlorogensäure, Salicylsäure, 2,3-Dihydroxybenzoesäure (Brenzcatechinsäure), 2,4-Dihydroxybenzoesäure (β-Resorcylsäure), 2,5-Dihydroxybenzoesäure (Gentisinsäure), 2,6-Dihydroxybenzoesäure (γ-Resorcylsäure), 3,4-Dihydroxybenzoesäure (Protocatechusäure), 3,5-Dihydroxybenzoesäure (α-Resorcylsäure), Gallussäure, 2-Cyano-3-phenylzimtsäure, den Methyl-, Ethyl- Isopropyl-, Propyl-, Butyl-, Hexyl-, Ethylhexyl-, Octyl-, Decyl-, Ethyloctyl-, Cetyl- und Stearylestern und den physiologisch verträglichen Salzen.
Erfindungsgemäß werden die aromatischen Carbonsäuren mit 6 - 24 Kohlenstoffatomen, 1 Carboxylgruppe, 1 - 2 Phenylresten, 1 - 6 Hydroxy- und/oder Cyanogruppen, deren Ester sowie deren physiologisch verträglichen Salze und ihre physiologisch verträglichen Salze in Mengen von 0,001 bis 10 Gew.-%, bevorzugt 0,005 bis 5 Gew.-% und besonders bevorzugt 0,01 bis 2 Gew.-%, jeweils bezogen auf die gesamte kosmetische Zusammensetzung, eingesetzt.

In einer weiteren bevorzugten Ausführungsform sind die erfindungsgemäß verwendeten C₂-C₁₁-Aminosäuren ausgewählt aus Asparaginsäure, Glutaminsäure, Pyroglutaminsäure, Lysin, Arginin, Histidin, Alanin, Valin, Leucin, Isoleucin, Prolin, Tryptophan, Phenylalanin, Methionin, Glycin, Serin, Tyrosin, Threonin, Cystein, Asparagin und Glutamin, deren N-C₂-C₂₂-Acylderivaten sowie deren physiologisch verträglichen Salzen. Die Aminosäuren können einzeln oder im Gemisch eingesetzt werden. Erfindungsgemäß geeignet sind insbesondere Aminosäuren-Gemische, die aus Pflanzen, insbesondere Getreidepflanzen, gewonnen wurden. Die genannten Aminosäuren können an der Aminogruppe mit einem C₂ - C₂₂-Acylrest derivatisiert sein, der bevorzugt ausgewählt ist aus einem Octanoyl-, Decanoyl-, Lauroyl-, Myristoyl-, Cetoyl-, Palmitoyl- oder Stearoyl-Rest. Besonders bevorzugt sind Aluminiumglycinat, Zinkglycinat, Zinkpyroglutamat, Natriumoctanoylglutamat, Natriumdecanoylglutamat, Natriumlauroylglutamat, Natriummyristoylglutamat, Natriumcetoylglutamat und Natriumstearoylglutamat und die Lauroyl-Derivate von aus Getreidepflanzen gewonnenen Aminosäuren.
Die Getreidepflanzen, aus denen die erfindungsgemäß geeigneten Aminosäuren gewonnen werden, unterliegen keiner Einschränkung. Geeignet sind beispielsweise Hafer, Weizen, Gerste und Roggen; besonders geeignet ist Hafer. Ein geeigneter Lipase-Inhibitor ist das Handelsprodukt Proteol OAT von der Firma Seppic.
Erfindungsgemäß werden die C₂-C₁₁-Aminosäuren, deren N-C₂-C₂₂-Acylderivate sowie deren physiologisch verträglichen Salze in Mengen von 0,001 bis 10 Gew.-%, bevorzugt 0,005 bis 5 Gew.-% und besonders bevorzugt 0,01 bis 2 Gew.-%, jeweils bezogen auf die gesamte kosmetische Zusammensetzung, eingesetzt.

Die physiologisch verträglichen Salze der vorgenannten Lipase-Inhibitoren sind ausgewählt aus den Ammonium-, Alkalimetall-, Magnesium-, Calcium-, Aluminium-, Zink- und Mangan-Salzen. Bevorzugt sind die Natrium-, Kalium-, Magnesium-, Aluminium-, Zink- und Mangan-Salze.

In einer weiteren bevorzugten Ausführungsform sind die erfindungsgemäß verwendeten Tetraester von Pentaerythrit mit C₂-C₄-Carbonsäuren, die einen substituierten Arylrest enthalten, ausgewählt aus Pentaerythrittetrakis[3(3,5-di-tert.-butyl-4-hydroxyphenyl)-propionat].
Erfindungsgemäß werden die Tetraester von Pentaerythrit mit C₂-C₄-Carbonsäuren, die einen substituierten Arylrest enthalten, in Mengen von 0,001 bis 10 Gew.-%, bevorzugt 0,005 bis 5 Gew.-% und besonders bevorzugt 0,01 bis 2 Gew.-%, jeweils bezogen auf die gesamte kosmetische Zusammensetzung, eingesetzt.

In einer weiteren besonders bevorzugten Ausführungsform sind die erfindungsgemäß verwendeten, gewünschtenfalls ethoxylierten Mono- und Diester von Glycerin und Polyglycerinen wie Diglycerin, Triglycerin und Tetraglycerin, mit gewünschtenfalls hydroxylierten C₈-C₂₂-Fettsäuren, insbesondere Caprylsäure, Caprinsäure, Laurinsäure, 2-Hydroxylaurinsäure, Myristinsäure, Palmitinsäure, Isohexadecansäure, Stearinsäure, 12-Hydroxystearinsäure oder Isostearinsäure, und gewünschtenfalls Ethoxylierungsgraden von 2 bis 100, bevorzugt 5 bis 80 und besonders bevorzugt 20 bis 60, ausgewählt aus PEG-20-glycerylstearat, Glycerylcaprylat, Polyglyceryl-2-dipolyhydroxystearat und Polyglyceryl-3-diisostearat.
Erfindungsgemäß werden die gewünschtenfalls ethoxylierten Mono- und Diester von Glycerin und Polyglycerinen mit gewünschtenfalls hydroxylierten C₈-C₂₂-Fettsäuren in Mengen von 0,001 bis 10 Gew.-%, bevorzugt 0,005 bis 5 Gew.-% und besonders bevorzugt 0,01 bis 2 Gew.-%, jeweils bezogen auf die gesamte kosmetische Zusammensetzung, eingesetzt.

In einer weiteren bevorzugten Ausführungsform sind die erfindungsgemäß verwendeten Ether und Ester von gewünschtenfalls alkylierten Mono-, Oligo- und Polysacchariden ausgewählt aus den Monoestern von C₆-C₂₂-Fettsäuren mit Glucose und Oligoglucose, den Estern von hydroxysubstituierten Bi- oder Tricarbonsäuren mit alkylierter Glucose, den Veretherungsprodukten von C₈-C₂₂-Alkoholen mit den aus dem Stroh oder der Kleie von Getreidepflanzen gewonnenen Glycosiden, sowie aus Chondroitinsulfat.
Bevorzugt verwendete Glucoseester sind zum einen die Monoester von C₆-C₂₂-Fettsäuren mit Glucose und Oligoglucose, wobei die C₆-C₂₂-Fettsäuren bevorzugt ausgewählt sind aus Decansäure, Laurinsäure, Myristinsäure, Palmitinsäure und Stearinsäure und die Glucose mit einem Oligomerisierungsgrad von 1,0 bis 1,5 vorliegt. Ein besonders bevorzugtes Beispiel ist APG-Monolaurat, ein Glucoselaurinsäuremonoester der Firma Cognis.
Weitere bevorzugt verwendete Glucoseester sind die Ester von hydroxysubstituierten Bi- oder Tricarbonsäuren mit alkylierter Glucose der allgemeinen Formel (I),

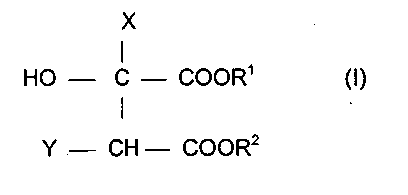

in der X ein Wasserstoff-Atom oder eine -CH₂COOR-Gruppe ist, Y ein Wasserstoff-Atom oder -OH-Gruppe ist unter der Bedingung, dass Y ein Wasserstoff-Atom ist, wenn X -CH₂COOR ist, R, R¹ und R² unabhängig voneinander ein Wasserstoffatom, ein Alkali- oder Erdalkalimetallkation, eine Ammoniumgruppe, das Kation einer ammoniumorganischen Base oder einen Rest Z bedeuten, wobei Z einen mit einem C₆-C₁₈-Alkanol oder mit technischen Mischungen von C₆-C₁₈-Alkanolen veretherten Glucose- oder Oligoglucoserest darstellt, mit der Bedingung, dass mindestens eine der Gruppen R, R¹ oder R² ein Rest Z ist. Solche Verbindungen sind z. B. in der Druckschrift EP 258 814 B1 beschrieben. Besonders bevorzugt verwendete Verbindungen der Formel (I) sind Dinatrium-Kokosalkylpolyglucosidcitrat (Disodium Cocopolyglucose Citrate, als Handelsprodukt Eucarol® AGE EC von der Firma Cesalpinia Chemicals erhältlich) und Dinatrium-Kokosalkylpolyglucosidtartrat (Disodium Cocopolyglucose Tartrate, als Handelsprodukt Eucarol® AGE ET von der Firma Cesalpinia Chemicals erhältlich).
Die Getreidepflanzen, aus denen erfindungsgemäß geeignete, mit C₈-C₂₂-Alkoholen veretherte Glycoside gewonnen werden können, unterliegen keiner Einschränkung. Geeignet sind beispielsweise Weizen, Hafer, Gerste und Roggen; besonders geeignet ist Weizen. Zur Veretherung werden C₈-C₂₂-Alkohole, insbesondere C₁₂-C₂₀-Alkohole und besonders bevorzugt C₁₆-C₁₈-Alkohole wie Cetylalkohol und Stearylalkohol sowie deren technische Mischungen verwendet.
Besonders bevorzugt geeignet sind die Handelsprodukte Xyliance und Emuliance von der Firma Soliance, die durch Veretherung von Cetearylalkohol mit den aus Weizenstroh oder Weizenkleie gewonnenen Glycosiden erhältlich sind.

Erfindungsgemäß werden die Ether und Ester von gewünschtenfalls alkylierten Mono-, Oligo- und Polysacchariden in Mengen von 0,01 bis 15 Gew.-%, bevorzugt 0,05 bis 10 Gew.-% und besonders bevorzugt 0,1 bis 3 Gew.-%, jeweils bezogen auf die gesamte kosmetische Zusammensetzung, eingesetzt.

In einer weiteren bevorzugten Ausführungsform sind die erfindungsgemäß verwendeten Pflanzenextrakte ausgewählt aus den Extrakten von Centella asiatica, Apfelkernen, den Früchten (Beeren) von Phyllanthus emblica, Meeresalgen, Henna, den Blättern des Olivenbaumes (Olea europaea), der Rinde des Afrikanischen Zwetschgenbaumes (Pygeum africanum, Prunus africana), Weidenröschen (Epilobium angustifolium, Willowherb), Papayasaft, Zitronen und Palmarosaöl (Cymbopogon Martini Oil).
Erfindungsgemäß werden die Pflanzenextrakte in Mengen von 0,001 bis 20 Gew.-%, bevorzugt 0,005 bis 10 und besonders bevorzugt 0,01 bis 5 Gew.-% Aktivsubstanz, jeweils bezogen auf die gesamte kosmetische Zusammensetzung, eingesetzt.

Da die erfindungsgemäß geeigneten Pflanzenextrakte Flavonoide enthalten, sind auch Flavonoide explizit als erfindungsgemäß geeignete und bevorzugte Lipase-Inhibitoren offenbart.
Die erfindungsgemäß bevorzugten Flavonoide umfassen die Glycoside der Flavone, der Flavanone, der 3-Hydroxyflavone (Flavonole), der Aurone und der Isoflavone. Besonders bevorzugte Flavonoide sind ausgewählt aus Apigenin-7-glucosid, α-Glucosylrutin, α-Glucosylmyricetin, α-Glucosylisoquercetin, α-Glucosylquercetin, Naringin (Aurantiin, Naringenin-7-rhamnoglucosid), Hesperidin (3',5,7-Trihydroxy-4'-methoxyflavanon-7-rhamnoglucosid, Hesperitin-7-O-rhamnoglucosid), Neohesperidin, Rutin (3,3',4',5,7-Pentahydroxyflavon-3-rhamnoglucosid, Quercetin-3-rhamnoglucosid), Troxerutin (3,5-Dihydroxy-3',4',7-tris(2-hydroxyethoxy)-flavon-3-(6-O-(6-deoxy-α-L-mannopyranosyl)-β-D-glucopyranosid)), Monoxerutin (3,3',4',5-Tetrahydroxy-7-(2-hydroxyethoxy)-flavon-3-(6-O-(6-deoxy-α-L-mannopyranosyl)-β-D-glucopyranosid)), Diosmin (3',4',7-Trihydroxy-5-methoxyflavanon-7-rhamnoglucosid) und Eriodictin.
Ebenfalls bevorzugt sind die aus zwei Flavonoideinheiten aufgebauten Biflavonoide, die z. B. in Gingko-Arten vorkommen. Weitere bevorzugte Flavonoide sind die Chalkone, vor allem Phlorizin und Neohesperidindihydrochalkon.
Erfindungsgemäß werden die Flavonoide in Mengen von 0,001 bis 1 Gew.-%, bevorzugt 0,005 bis 0,5 Gew.-% und besonders bevorzugt 0,01 bis 0,4 Gew.-%, jeweils bezogen auf die Flavonoidaktivsubstanz in der gesamten kosmetischen Zusammensetzung, eingesetzt.

In einer weiteren bevorzugten Ausführungsform sind die erfindungsgemäß verwendeten Lipase-Inhibitoren aus Polyphenolen ausgewählt. Unter Polyphenolen sind erfindungsgemäß aromatische Verbindungen zu verstehen, die mindestens zwei phenolische Hydroxy-Gruppen im Molekül enthalten. Hierzu zählen die drei Dihydroxybenzole Brenzcatechin, Resorcin und Hydrochinon, weiterhin Phloroglucin, Pyrogallol und Hexahydroxybenzol. In der Natur treten freie und veretherte Polyphenole beispielsweise in Blütenfarbstoffen (Anthocyanidine, Flavone), in Gerbstoffen (Catechine, Tannine), als Flechten- oder Farn-Inhaltsstoffe (Usninsäure, Acylpolyphenole), in Ligninen und als Gallussäure-Derivate auf. Bevorzugte Polyphenole sind Flavone, Catechine, Usninsäure, und als Tannine die Derivate der Gallussäure, Digallussäure und Digalloylgallussäure. Besonders bevorzugte Polyphenole sind die monomeren Catechine, das heißt die Derivate der Flavan-3-ole, und Leukoanthocyanidine, das heißt die Derivate der Leukoanthocyanidine, die bevorzugt in 5,7,3',4',5'-Stellung phenolische Hydroxygruppen tragen, bevorzugt Epicatechin und Epigallocatechin, sowie die daraus durch Selbstkondensation entstehenden Gerbstoffe. Solche Gerbstoffe werden bevorzugt nicht in isolierter Reinsubstanz, sondern als Extrakte gerbstoffreicher Pflanzenteile eingesetzt, z. B. Extrakte von Catechu, Quebracho, Pinienrinde und Eichenrinde sowie anderer Baumrinden, Blättern von Grünem Tee (camellia sinensis) und Mate. Ebenfalls besonders bevorzugt sind die Tannine.
Erfindungsgemäß werden die Polyphenoie in Mengen von 0,001 bis 10 Gew.-%, bevorzugt 0,005 bis 5 Gew.-% und besonders bevorzugt 0,08 bis 3 Gew.-%, jeweils bezogen auf die gesamte kosmetische Zusammensetzung, eingesetzt.

In einer weiteren bevorzugten Ausführungsform sind die erfindungsgemäß verwendeten Lipase-Inhibitoren ausgewählt aus Ubichinonen der Formel (II), 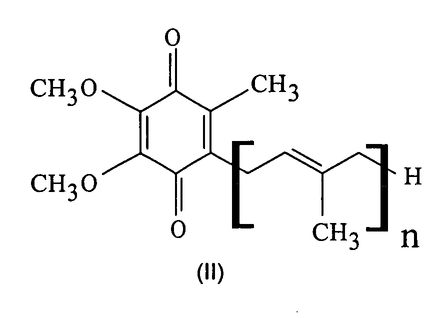 mit n = 6, 7, 8, 9 oder 10.
Besonders bevorzugt ist das Ubichinon der Formel (II) mit n = 10, auch bekannt als Coenzym Q10.

Erfindungsgemäß werden die Ubichinone in Mengen von 0,0001 bis 1 Gew.-%, bevorzugt 0,001 bis 0,5 Gew.-% und besonders bevorzugt 0,005 bis 0,1 Gew.-%, jeweils bezogen auf die gesamte kosmetische Zusammensetzung, eingesetzt.

In einer weiteren bevorzugten Ausführungsform sind die erfindungsgemäß verwendeten Lipase-Inhibitoren ausgewählt aus 2-(2H-Benzotriazol-2-yl)-6-alkylphenolderivaten der Strukturformel (III),

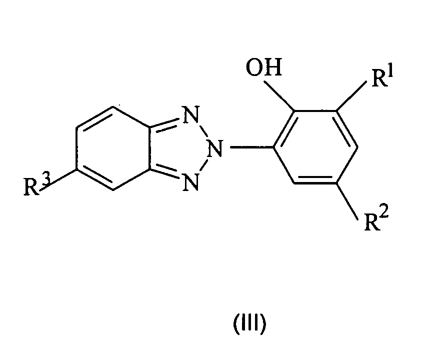

in der R¹ eine lineare oder verzweigte C₁-C₁₂-Alkylgruppe,
R² eine lineare oder verzweigte C₁-C₆-Alkylgruppe oder eine Sulfonsäuregruppe -SO₃X mit X = Wasserstoffatom oder ein einwertiges, physiologisch verträgliches Kation, und R³ ein Atom H, F, Cl oder Br darstellen.

Die bevorzugt verwendeten 2-(2H-Benzotriazol-2-yl)-6-alkylphenolderivate enthalten den Benzotriazolyl-Rest in ortho-Position zur Hydroxygruppe. Besonders bevorzugt werden solche Verbindungen verwendet, die in der zweiten ortho-Position eine C₁-C₁₂-Alkylgruppe aufweisen. Die C₁-C₁₂-Alkylgruppe sind dabei ausgewählt aus Methyl-, Ethyl-, n-Propyl-, Isopropyl-, n-Butyl-, sec-Butyl- tert-Butyl-, n-Pentyl-, 1-Methylbutyl-, 2-Methylbutyl-, 3-Methylbutyl-, Neopentyl-, n-Hexyl-, 1-Methylpentyl-, 2-Methylpentyl-, 3-Methylpentyl-, 4-Methylpentyl-, 1,1-Dimethylbutyl-, 1,2-Dimethylbutyl-, 1,3-Dimethylbutyl-, 2,2-Dimethylbutyl-, 2,3-Dimethylbutyl-, 3,3-Dimethylbutyl-, n-Heptyl-, iso-Heptyl- mit verschiedenen Methyl- und Ethylverzweigungen, n-Octyl-, iso-Octyl-Gruppen mit verschiedenen Methyl-, Ethyl-, Propyl- und Butylverzweigungen, n-Nonyl-, iso-Nonyl-Gruppen mit verschiedenen Methyl-, Ethyl-, Propyl-, Butyl- und Pentylverzweigungen, n-Decyl-, iso-Decyl-Gruppen mit verschiedenen Methyl-, Ethyl-, Propyl-, Butyl-, Pentyl- und Hexylverzweigungen, n-Undecyl-, iso-Undecyl-Gruppen mit verschiedenen Methyl-, Ethyl-, Propyl-, Butyl-, Pentyl-, Hexyl- und Heptylverzweigungen, n-Dodecyl- und iso-Dodecyl-Gruppen mit verschiedenen Methyl-, Ethyl-, Propyl-, Butyl-, Pentyl-, Hexyl- und Heptylverzweigungen. Erfindungsgemäß geeignete Kationen X sind alle physiologisch verträglichen Kationen, beispielsweise Natrium, Kalium und Ammonium. Besonders bevorzugt verwendet werden das 3-(2H-Benzotriazol-2-yl)-5-sec-butyl-4-phenolsulfonsäure-natriumsalz, als Handelsprodukte Tinogard™ HS (Reinsubstanz) oder Cibafast™ H (Compound mit Buteth-3 und Tributylcitrat) von Ciba erhältlich, 2-(2H-Benzotriazol-2-yl)-6-dodecyl-4-methylphenol, als Tinogard™ TL von Ciba erhältlich, und 2-(5-Chloro-2H-benzotriazol-2-yl)-4-methyl-6-t-butylphenol, als Tinogard™ AS von Ciba erhältlich.
Erfindungsgemäß werden die 2-(2H-Benzotriazol-2-yl)-6-alkylphenolderivate in Mengen von 0,001 bis 10 Gew.-%, bevorzugt 0,005 bis 5 Gew.-% und besonders bevorzugt 0,01 bis 2 Gew.-%, jeweils bezogen auf die gesamte kosmetische Zusammensetzung, eingesetzt.

In einer weiteren bevorzugten Ausführungsform wird der erfindungsgemäß verwendete Lipase-inhibitor α-Bisabolol in Mengen von 0,001 bis 5 Gew.-%, bevorzugt 0,01 bis 2 Gew.-% und besonders bevorzugt 0,1 bis 1 Gew.-%, jeweils bezogen auf die gesamte kosmetische Zusammensetzung, eingesetzt.

In einer weiteren bevorzugten Ausführungsform wird der erfindungsgemäß verwendete Lipase-Inhibitor 2,2-Dimethyl-3-phenyl-1-propanol in Mengen von 0,001 bis 5 Gew.-%, bevorzugt 0,01 bis 2 Gew.-% und besonders bevorzugt 0,1 bis 1 Gew.-%, jeweils bezogen auf die gesamte kosmetische Zusammensetzung, eingesetzt.

In einer weiteren bevorzugten Ausführungsform sind die erfindungsgemäß verwendeten Lipase-Inhibitoren ausgewählt aus Papain (Papaya-Petidase I, EC 3.4.22.2), Chymopapain (Papaya-Petidase II, EC 3.4.22.6), Bromelain (EC 3.4.22.32 und EC 3.4.22.33), Ficin (Ficain, EC 3.4.22.3) und Asclepain (EC 3.4.22.7). Hierbei handelt es sich um pflanzliche Peptidasen, die aus Papaya, Ananas, Feigen und Asclepia speciosa erhältlich sind. Sie werden erfindungsgemäß in Mengen von 0,00001 bis 1 Gew.-%, bevorzugt 0,001 bis 0,5 Gew.-% und besonders bevorzugt 0,01 bis 0,1 Gew.-%, jeweils bezogen auf die gesamte kosmetische Zusammensetzung, eingesetzt. Besonders bevorzugt ist, diese Peptidasen nicht in Reinform einzusetzen, sondern Peptidasenhaltige pflanzliche Extrakte zu verwenden. Als erfindungsgemäß besonders geeignet erwiesen hat sich das Handelsprodukt Xyleine, ein Extrakt aus Papayasaft, Zitrone und Olivenblättern.

In einer weiteren bevorzugten Ausführungsform werden die erfindungsgemäß als Lipase-Inhibitoren verwendeten Phenylpropyldimethylsiloxysilicate, erhältlich als Handelsprodukt Baysilone CF-1301 der Firma GE Silicones, in Mengen von 0,1 bis 10 Gew.-%, bevorzugt 0,5 bis 5 Gew.-% und besonders bevorzugt 1,0 bis 5 Gew.-%, jeweils bezogen auf den Anteil des Handelsproduktes an der gesamten kosmetischen Zusammensetzung, eingesetzt.

Erfindungsgemäß bevorzugt ist weiterhin die Verwendung von Aluminiumchlorohydrat als Lipase-Inhibitor. Aluminiumchlorohydrat ist ein gebräuchlicher Antitranspirant-Wirkstoff, der als Adstringens allerdings in höheren Konzentrationen von 5 bis 25 Gew.-% eingesetzt werden muss, um in für den Verbraucher akzeptabler Weise wirksam zu sein. Für die Inhibierung von Lipase genügen dagegen niedrigere Konzentrationen von 0,2 bis 3 Gew.-%, bevorzugt 0,5 bis 3 Gew.-% und besonders bevorzugt 1,0 bis 2,0 Gew.-%, jeweils bezogen auf die gesamte kosmetische Zusammensetzung.

Als erfindungsgemäß geeignet hat sich weiterhin die Verwendung des Rohstoffs Protectate HR, einem Riechstoffgemisch erhältlich von Haarmann und Reimer, erwiesen. Protectate HR wird erfindungsgemäß als Lipase-Inhibitor in Mengen von 0,001 bis 5 Gew.-%, bevorzugt 0,01 bis 2 Gew.-% und besonders bevorzugt 0,1 bis 1 Gew.-%, jeweils bezogen auf die gesamte kosmetische Zusammensetzung, eingesetzt.

Ein weiterer Gegenstand der vorliegenden Erfindung ist ein Verfahren zur Verringerung von Körpergeruch mittels Inhibierung von Lipase auf der Haut, das dadurch gekennzeichnet ist, dass eine kosmetische Deodorant- oder Antitranspirant-Zusammensetzung, enthaltend mindestens eine Lipase-inhibierende Substanz, ausgewählt aus einbasigen C₂-C₈-Carbonsäuren mit 1 - 7 Hydroxygruppen und deren physiologisch verträglichen Salzen,
aromatischen Carbonsäuren mit 6 - 24 Kohlenstoffatomen, 1 Carboxylgruppe, 1 - 2 Phenylresten, 1 - 6 Hydroxy- und/oder Cyanogruppen sowie deren physiologisch verträglichen Salzen,
C₂-C₁₁-Aminosäuren, deren N-C₂-C₂₂-Acylderivaten sowie deren physiologisch verträglichen Salzen,
den Tetraestern von Pentaerythrit mit C₂-C₄-Carbonsäuren, die einen substituierten Arylrest enthalten,
gewünschtenfalls ethoxylierten Mono- und Diestern von Glycerin und Polyglycerinen mit gewünschtenfalls hydroxylierten C₈-C₂₂-Fettsäuren,
den Ethern und Estern von gewünschtenfalls alkylierten Mono-, Oligo- und Polysacchariden,
Pflanzenextrakten, ausgewählt aus den Extrakten von Centella asiatica, Apfelkernen, den Früchten (Beeren) von Phyllanthus emblica, Meeresalgen, Henna, den Blättern des Olivenbaumes (Olea europaea), der Rinde des Afrikanischen Zwetschgenbaumes (Pygeum africanum, Prunus africana), Weidenröschen (Epilobium angustifolium, Willowherb), Papayasaft, Zitronen und Palmarosaöl (Cymbopogon Martini Oil), Flavonoiden, Polyphenolen, Ubichinonen, 2-(2H-Benzotriazol-2-yl)-6-alkylphenolderivaten, α-Bisabolol, 2,2-Dimethyl-3-phenyl-1-propanol, Papain (Papaya-Petidase I, EC 3.4.22.2), Chymopapain (Papaya-Petidase II, EC 3.4.22.6), Bromelain (EC 3.4.22.32 und EC 3.4.22.33), Ficin (Ficain, EC 3.4.22.3) und Asclepain (EC 3.4.22.7), Phenylpropyldimethylsiloxysilicaten sowie Aluminiumchlorohydrat,
auf die Haut, insbesondere auf die Haut der Achselhöhlen, aufgetragen wird.

Die kosmetischen Deodorant- oder Antitranspirant-Zusammensetzungen, die die erfindungsgemäß verwendeten Lipase-Inhibitoren enthalten, können als Puder, in Stiftform, als Aerosolspray, Pumpspray, flüssige und gelförmige Roll on-Applikation, Creme, Gel und als getränktes flexibles Substrat vorliegen.
Deodorant- oder Antitranspirant-Stifte können in gelierter Form, auf wasserfreier Wachsbasis und auf Basis von W/O-Emulsionen und O/W-Emulsionen vorliegen. Gelstifte können auf der Basis von Fettsäureseifen, Dibenzylidensorbitol, N-Acylaminosäureamiden, 12-Hydroxystearinsäure und anderen Gelbildnern hergestellt werden.
Aerosolsprays, Pumpsprays, Roll on-Applikationen und Cremes können als Wasser-in-Öl-Emulsion, Öl-in-Wasser-Emulsion, Siliconöl-in-Wasser-Emulsion, Wasser-in-Öl-Mikroemulsion, Öl-in-Wasser-Mikroemulsion, Siliconöl-in-Wasser-Mikroemulsion, wasserfreie Suspension, alkoholische und hydroalkoholische Lösung, wässriges Gel und als Öl vorliegen. Alle genannten Zusammensetzungen können verdickt sein, beispielsweise auf der Basis von Fettsäureseifen, Dibenzylidensorbitol, N-Acylaminosäureamiden, 12-Hydroxystearinsäure, Polyacrylaten vom Carbomer- und Carbopol-Typ, Polyacrylamiden und Polysacchariden, die chemisch und/oder physikalisch modifiziert sein können.
Die Emulsionen und Mikroemulsionen können transparent, translucent oder opak sein.

Die kosmetischen Deodorant- oder Antitranspirant-Zusammensetzungen, die die erfindungsgemäß verwendeten Lipase-Inhibitoren enthalten, können weiterhin Fettstoffe enthalten. Unter Fettstoffen sind Fettsäuren, Fettalkohole, natürliche und synthetische kosmetische Ölkomponenten sowie natürliche und synthetische Wachse zu verstehen, die sowohl in fester Form als auch flüssig in wässriger oder öliger Dispersion vorliegen können.
Als Fettsäuren können eingesetzt werden lineare und/oder verzweigte, gesättigte und/oder ungesättigte C₈₋₃₀-Fettsäuren. Bevorzugt sind C₁₀₋₂₂-Fettsäuren. Beispiele sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachidonsäure, Gadoleinsäure, Behensäure und Erucasäure sowie deren technische Mischungen. Besonders bevorzugt ist der Einsatz von Stearinsäure. Die eingesetzten Fettsäuren können eine oder mehrere Hydroxygruppen tragen. Bevorzugte Beispiele hierfür sind die α-Hydroxy-C₈-C₁₈-Carbonsäuren sowie 12-Hydroxystearinsäure.
Die Einsatzmenge beträgt dabei 0,1 - 15 Gew.-%, bevorzugt 0,5 - 10 Gew.-%, besonders bevorzugt 1 - 5 Gew.-%, jeweils bezogen auf die gesamte Zusammensetzung.
Als Fettalkohole können eingesetzt werden gesättigte, ein- oder mehrfach ungesättigte, verzweigte oder unverzweigte Fettalkohole mit 6 - 30, bevorzugt 10 - 22 und ganz besonders bevorzugt 12 - 22 Kohlenstoffatomen. Einsetzbar im Sinne der Erfindung sind z.B. Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole.
Für Stiftformulierungen werden häufig Wachse verwendet. Als natürliche oder synthetische Wachse können erfindungsgemäß eingesetzt werden feste Paraffine oder Isoparaffine, Pflanzenwachse wie Candelillawachs, Carnaubawachs, Espartograswachs, Japanwachs, Korkwachs, Zuckerrohrwachs, Ouricurywachs, Montanwachs, Sonnenblumenwachs, Fruchtwachse und tierische Wachse, wie z. B. Bienenwachse und andere Insektenwachse, Walrat, Schellackwachs, Wollwachs und Bürzelfett, weiterhin Mineralwachse, wie z. B. Ceresin und Ozokerit oder die petrochemischen Wachse, wie z. B. Petrolatum, Paraffinwachse, Microwachse aus Polyethylen oder Polypropylen und Polyethylenglycolwachse. Es kann vorteilhaft sein, hydrierte oder gehärtete Wachse einzusetzen. Weiterhin sind auch chemisch modifizierte Wachse, insbesondere die Hartwachse, z. B. Montanesterwachse, Sasolwachse und hydrierte Jojobawachse, einsetzbar.
Weiterhin geeignet sind die Triglyceride gesättigter und gegebenenfalls hydroxylierter C₁₆₋₃₀-Fettsäuren, wie z. B. gehärtete Triglyceridfette (hydriertes Palmöl, hydriertes Kokosöl, hydriertes Rizinusöl), Glyceryltribehenat oder Glyceryltri-12-hydroxystearat, weiterhin synthetische Vollester aus Fettsäuren und Glykolen (z. B. Syncrowachs®) oder Polyolen mit 2 - 6 C-Atomen, Fettsäuremonoalkanolamide mit einem C₁₂₋₂₂-Acylrest und einem C₂₋₄-Alkanolrest, Ester aus gesättigten und/oder ungesättigten, verzweigten und/oder unverzweigten Alkancarbonsäuren einer Kettenlänge von 1 bis 80 C-Atomen und gesättigten und/oder ungesättigten, verzweigten und/oder unverzweigten Alkoholen einer Kettenlänge von 1 bis 80 C-Atomen, darunter z. B. synthetische Fettsäure-Fettalkoholester wie Stearylstearat oder Cetylpalmitat, Ester aus aromatischen Carbonsäuren, Dicarbonsäuren bzw. Hydroxycarbonsäuren (z. B. 12-Hydroxystearinsäure) und gesättigten und/oder ungesättigten, verzweigten und/ oder unverzweigten Alkoholen einer Kettenlänge von 1 bis 80 C-Atomen, Lactide langkettiger Hydroxycarbonsäuren und Vollester aus Fettalkoholen und Di- und Tricarbonsäuren, z. B. Dicetylsuccinat oder Dicetyl-/stearyladipat, sowie Mischungen dieser Substanzen, sofern die einzelnen Wachskomponenten oder ihre Mischung bei Raumtemperatur fest sind. Besonders bevorzugt ist, die Wachskomponenten zu wählen aus der Gruppe der Ester aus gesättigten, unverzweigten Alkancarbonsäuren einer Kettenlänge von 14 bis 44 C-Atomen und gesättigten, unverzweigten Alkoholen einer Kettenlänge von 14 bis 44 C-Atomen, sofern die Wachskomponente oder die Gesamtheit der Wachskomponenten bei Raumtemperatur fest sind. Insbesondere vorteilhaft können die Wachskomponenten aus der Gruppe der C₁₆₋₃₆-Alkylstearate, der C₁₀₋₄₀-Alkylstearate, der C₂₋₄₀-Alkylisostearate, der C₂₀₋₄₀-Dialkylester von Dimersäuren, der C₁₈₋₃₈-Alkylhydroxystearoylstearate, der C₂₀₋₄₀-Alkylerucate gewählt werden, ferner sind C₃₀₋₅₀-Alkylbienenwachs sowie Cetearylbehenat einsetzbar. Auch Silikonwachse, zum Beispiel Stearyltrimethylsilan/Stearylalkohol sind gegebenenfalls vorteilhaft. Besonders bevorzugte Wachskomponenten sind die Ester aus gesättigten, einwertigen C₂₀-C₆₀-Alkoholen und gesättigten C₈-C₃₀-Monocarbonsäuren, insbesondere ein C₂₀-C₄₀-Alkylstearat bevorzugt, das unter dem Namen Kesterwachs® K82H von der Firma Koster Keunen Inc. erhältlich ist. Das Wachs oder die Wachskomponenten sollten bei 25° C fest sein, jedoch im Bereich von 35 - 95°C schmelzen, wobei ein Bereich von 45 - 85 °C bevorzugt ist. Natürliche, chemisch modifizierte und synthetische Wachse können alleine oder in Kombination eingesetzt werden.
Die Wachskomponenten sind in einer Menge von 0,1 bis 40 Gew.-%, bezogen auf die Gesamtzusammensetzung, vorzugsweise 1 bis 30 Gew.-% und insbesondere 5 - 15 Gew.-% enthalten.
Die erfindungsgemäßen Zusammensetzungen können weiterhin wenigstens ein unpolares oder polares flüssiges Öl, das natürlich oder synthetisch sein kann, enthalten.
Die polare Ölkomponente kann ausgewählt sein aus pflanzlichen Ölen, z. B. Sonnenblumenöl, Olivenöl, Sojaöl, Rapsöl, Mandelöl, Jojobaöl und den flüssigen Anteilen des Kokosöls sowie synthetischen Triglyceridölen, aus Esterölen, das heißt den Estern von C₆-₃₀-Fettsäuren mit C₂₋₃₀-Fettalkoholen, aus Dicarbonsäureestern wie Di-n-butyladipat, Di-(2-ethylhexyl)-adipat und Di-(2-ethylhexyl)-succinat sowie Diolestern wie Ethylenglykoldioleat und Propylenglykoldi(2-ethylhexanoat), aus symmetrischen, unsymmetrischen oder cyclischen Estern der Kohlensäure mit Fettalkoholen, beispielsweise beschrieben in der DE-OS 197 56 454, Glycerincarbonat oder Dicaprylylcarbonat (Cetiol® CC), aus Mono,- Di- und Trifettsäureestern von gesättigten und/oder ungesättigten linearen und/oder verzweigten Fettsäuren mit Glycerin, z. B. Cutina® MD, aus verzweigten Alkanolen, z. B. Guerbet-Alkoholen mit einer einzigen Verzweigung am Kohlenstoffatom 2 wie 2-Hexyldecanol, 2-Octyldodecanol, Isotridecanol und Isohexadecanol, aus Alkandiolen, z. B. den aus Epoxyalkanen mit 12 - 24 C-Atomen durch Ringöffnung mit Wasser erhältlichen vicinalen Diolen, aus Etheralkoholen, z. B. den Monoalkylethern des Glycerins, des Ethylenglycols, des 1,2-Propylenglycols oder des 1,2-Butandiols, aus Dialkylethern mit jeweils 12 - 24 C-Atomen, z. B. den Alkyl-methylethern oder Di-n-alkylethern mit jeweils insgesamt 12 - 24 C-Atomen, insbesondere Di-n-octylether (Cetiol®OE ex Cognis), sowie aus Anlagerungsprodukten von Ethylenoxid und/oder Propylenoxid an ein- oder mehrwertige C₃₋₂₀-Alkanole wie Butanol und Glycerin, z. B. PPG-3-Myristylether (Witconol® APM), PPG-14-Butylether (Ucon Fluid® AP), PPG-15-Stearylether (Arlamol® E), PPG-9-Butylether (Breox® B25) und PPG-10-Butandiol (Macol® 57).
Die unpolare Ölkomponente kann ausgewählt sein aus flüssigen Paraffinölen, Isoparaffinölen, z. B. Isohexadecan und Isoeicosan, aus synthetischen Kohlenwasserstoffen, z. B. 1,3-Di-(2-ethyl-hexyl)-cyclohexan (Cetiol® S), sowie aus flüchtigen und nichtflüchtigen Siliconölen, die cyclisch, wie z. B. Decamethylcyclopentasiloxan und Dodecamethylcyclohexasiloxan, oder linear sein können, z. B. lineares Dimethylpolysiloxan, im Handel erhältlich z. B. unter der Bezeichnung Dow Corning® 190, 200, 244, 245, 344 oder 345 und Baysilon® 350 M.

Die erfindungsgemäßen Zusammensetzungen können weiterhin wenigstens einen wasserlöslichen Alkohol enthalten. Unter Wasserlöslichkeit versteht man erfindungsgemäß, dass sich wenigstens 5 Gew.-% des Alkohols bei 20 °C klar lösen oder aber ― im Falle langkettiger oder polymerer Alkohole - durch Erwärmen der Lösung auf 50 °C bis 60 °C in Lösung gebracht werden können. Geeignet sind je nach Darreichungsform einwertige Alkohole wie z. B. Ethanol, Propanol oder Isopropanol. Weiterhin geeignet sind wasserlösliche Polyole. Hierzu zählen wasserlösliche Diole, Triole und höherwertige Alkohole sowie Polyethylenglycole. Unter den Diolen eignen sich C₂-C₁₂-Diole, insbesondere 1,2-Propylenglycol, Butylenglycole wie z. B. 1,2-Butylenglycol, 1,3-Butylenglycol und 1,4-Butylenglycol, Hexandiole wie z. B. 1,6-Hexandiol. Weiterhin bevorzugt geeignet sind Glycerin und insbesondere Diglycerin und Triglycerin, 1,2,6-Hexantriol sowie die Polyethylenglycole (PEG) PEG-400, PEG-600, PEG-1000, PEG-1550, PEG-3000 und PEG-4000.
Die Menge des Alkohols oder des Alkohol-Gemisches in den erfindungsgemäßen Zusammensetzungen beträgt 1 - 50 Gew.-% und vorzugsweise 5 - 40 Gew.-%, bezogen auf die gesamte Zusammensetzung. Erfindungsgemäß kann sowohl ein Alkohol als auch ein Gemisch mehrerer Alkohole eingesetzt werden.

Die erfindungsgemäßen Zusammensetzungen können im wesentlichen wasserfrei sein, das heißt maximal 5 Gew.-%, bevorzugt maximal 1 Gew.-% Wasser enthalten. In wasserhaltigen Darreichungsformen beträgt der Wassergehalt 5 - 98 Gew.-%, bevorzugt 10 - 90 und besonders bevorzugt 15 - 85 Gew.-%, bezogen auf die gesamte Zusammensetzung.

Die erfindungsgemäßen Zusammensetzungen können weiterhin wenigstens ein hydrophil modifiziertes Silicon enthalten. Sie ermöglichen die Formulierung hochtransparenter Zusammensetzungen, reduzieren die Klebrigkeit und hinterlassen ein frisches Hautgefühl. Unter hydrophil modifizierten Siliconen werden erfindungsgemäß Polyorganosiloxane mit hydrophilen Substituenten verstanden, welche die Wasserlöslichkeit der Silicone bedingen. Erfindungsgemäß wird unter Wasserlöslichkeit verstanden, dass sich wenigstens 2 Gew.-% des mit hydrophilen Gruppen modifizierten Silicons in Wasser bei 20 °C lösen. Entsprechende hydrophile Substituenten sind beispielsweise Hydroxy-, Polyethylenglycol- oder Polyethylenglycol/Polypropylenglycol-Seitenketten sowie ethoxylierte Ester-Seitenketten. Erfindungsgemäß bevorzugt geeignet sind hydrophil modifizierte Silicon-Copolyole, insbesondere Dimethicone-Copolyole, die beispielsweise von Wacker-Chemie unter der Bezeichnung Belsil® DMC 6031, Belsil® DMC 6032, Belsil® DMC 6038 oder Belsil® DMC 3071 VP bzw. von Dow Corning unter der Bezeichnung DC 2501 im Handel sind. Besonders bevorzugt geeignet ist die Verwendung von Belsil® DMC 6038, da es die Formulierung hochtransparenter Zusammensetzungen ermöglicht, die beim Verbraucher eine höhere Akzeptanz erreichen. Erfindungsgemäß kann auch ein beliebiges Gemisch dieser Silicone eingesetzt werden.
Die Menge des hydrophil modifizierten Silicons oder des Alkohol-Gemisches in den erfindungsgemäßen Zusammensetzungen beträgt 0,5 - 10 Gew.-%, bevorzugt 1 - 8 Gew.-% und besonders bevorzugt 2 - 6 Gew.-%, bezogen auf das Gesamtgewicht der Zusammensetzung.

Die erfindungsgemäßen Zusammensetzungen können weiterhin wenigstens ein wasserlösliches Tensid enthalten. Als wasserlösliche Tenside eignen sich grundsätzlich alle in dem System zu 1 Gew.-% bei 20°C löslichen und in Wasser bei 20°C zu mindestens 1 Gew.-% löslichen Tenside. Obwohl die Struktur und lonogenität an sich unerheblich sind, scheinen nichtionische Tenside, insbesondere die bei Normaltemperatur (20°C) festen Anlagerungsprodukte des Ethylenoxids an Fettstoffmoleküle mit wenigstens einer alkoxylierbaren Gruppe, bevorzugt geeignet zu sein. Solche geeigneten Tenside sind z.B. die Anlagerungsprodukte von 10 - 40 Mol Ethylenoxid an lineare Fettalkohole mit 16 - 22 C-Atomen, an Fettsäuren mit 12 - 22 C-Atomen, an Fettsäurealkanolamide, an Fettsäuremonoglyceride, an Sorbitan-Fettsäuremonoester, an Fettsäurealkanolamide, an Fettsäureglyceride, z.B. an gehärtetes Rizinusöl, an Methylglucosidmonofettsäureester und Gemische davon.

Die erfindungsgemäßen Zusammensetzungen enthalten in einer bevorzugten Ausführungsform mindestens einen Antitranspirant-Wirkstoff. Als Antitranspirant-Wirkstoffe eignen sich wasserlösliche adstringierende metallische Salze, insbesondere anorganische und organische Salze des Aluminiums, Zirkoniums und Zinks bzw. beliebige Mischungen dieser Salze. Erfindungsgemäß wird unter Wasserlöslichkeit eine Löslichkeit von wenigstens 5 g Aktivsubstanz pro 100 g Lösung bei 20 °C verstanden. Erfindungsgemäß verwendbar sind beispielsweise Alaun (KAl(SO₄)₂ · 12 H₂O), Aluminiumsulfat, Aluminiumlactat, Natrium-Aluminium-Chlorhydroxylactat, Aluminiumchlorhydroxyallantoinat, Aluminiumchlorohydrat, Aluminiumsulfocarbolat, Aluminium-Zirkonium-Chlorohydrat, Zinkchlorid, Zinksulfocarbolat, Zinksulfat, Zirkoniumchlorohydrat und Aluminium-Zirkonium-Chlorohydrat-Glycin-Komplexe. Bevorzugt enthalten die Zusammensetzungen ein adstringierendes Aluminiumsalz, insbesondere Aluminiumchlorohydrat, und/oder eine Aluminium-Zirkonium-Verbindung. Die Antitranspirant-Wirkstoffe werden bei wässrigen Applikationen als wässrige Lösungen eingesetzt. In wasserfreien Zusammensetzungen werden die Antitranspirant-Wirkstoffe in fester Form eingesetzt. Sie sind in den erfindungsgemäßen Zusammensetzungen in einer Menge von 1 - 40 Gew.-%, vorzugsweise 5 - 30 Gew.-% und insbesondere 10 - 25 Gew.-% enthalten (bezogen auf die Menge der Aktivsubstanz in der Gesamtzusammensetzung). Aluminiumchlorohydrate werden beispielsweise pulverförmig als Micro Dry® Ultrafine von Reheis, als Chlorhydrol®, in aktivierter Form als Reach® 501 von Reheis sowie in Form einer wäßrigen Lösung als Locron® L von Clariant vertrieben. Unter der Bezeichnung Reach® 301 wird ein Aluminiumsesquichlorohydrat von Reheis angeboten. Auch die Verwendung von Aluminium-Zirkonium-Tetrachlorohydrex-Glycin-Komplexen, die beispielsweise von Reheis unter der Bezeichnung Rezal® 36G im Handel sind, ist erfindungsgemäß besonders vorteilhaft.

Weiterhin können die erfindungsgemäßen Zusammensetzungen keimhemmende oder desodorierende Wirkstoffe enthalten. Geeignet sind insbesondere Organohalogenverbindungen sowie -halogenide, quartäre Ammoniumverbindungen, eine Reihe von Pflanzenextrakten und Zinkverbindungen. Bevorzugt sind Chlorhexidin und Chlorhexidingluconat, Benzalkoniumhalogenide und Cetylpyridiniumchlorid. Desweiteren sind Natriumbicarbonat, Natriumphenolsulfonat und Zinkphenolsulfonat sowie z. B. die Bestandteile des Lindenblütenöls einsetzbar.
Weitere antibakteriell wirksame Deodorans-Wirkstoffe sind Lantibiotika, Glycoglycerolipide, Sphingolipide (Ceramide), Sterine und andere Wirkstoffe, die die Bakterienadhäsion an der Haut inhibieren, z. B. Glycosidasen, Lipasen, Proteasen, Kohlenhydrate, Di- und Oligosaccharidfettsäureester sowie alkylierte Mono- und Oligosaccharide.
Flüssige und gelförmige Darreichungsformen können Verdickungsmittel enthalten, z. B. Celluloseether, wie Hydroxypropylcellulose, Hydroxyethylcellulose und Methylhydroxypropylcellulose, verdickende Polymere auf Basis von Polyacrylaten, die gewünschtenfalls vernetzt sein können, z. B. die Carbopoltypen oder Pemulen®-Produkte, oder auf Basis von Polyacrylamiden oder sulfonsäuregruppenhaltigen Polyacrylaten, z. B Sepigel® 305 oder Simulgel® EG, weiterhin anorganische Verdicker, z. B. Bentonite und Hectorite (Laponite®).
Die erfindungsgemäßen Zusammensetzungen können weitere kosmetisch und dermatologisch wirksame Stoffe enthalten, wie beispielsweise entzündungshemmende Substanzen, Feststoffe, ausgewählt aus Kieselsäuren, z. B. Aerosil®-Typen, Kieselgelen, Siliciumdioxid, Tonen, z. B. Bentonite oder Kaolin, Magnesiumaluminiumsilikaten, z. B. Talkum, Bornitrid, Titandioxid, das gewünschtenfalls beschichtet sein kann, gegebenenfalls modifizierten Stärken und Stärkederivaten, Cellulosepulvern und Polymerpulvern, desweiteren Pflanzenextrakte, Proteinhydrolysate, Vitamine, Parfümöle, Sebostatika, Anti-Akne-Wirkstoffe sowie Keratolytika.

Die kosmetischen Deodorant- oder Antitranspirant-Zusammensetzungen, die die erfindungsgemäß verwendeten Lipase-Inhibitoren enthalten, können, soweit sie flüssig vorliegen, auf flexible und saugfähige Träger aufgebracht und als Deodorant- oder Antitranspirant-Tücher oder Schwämmchen angeboten werden. Als flexible und saugfähige Träger im Sinne der Erfindung eignen sich z. B. Träger aus Textilfasern, Kollagen oder polymeren Schaumstoffen. Als Textilfasern können sowohl Naturfasern wie Cellulose (Baumwolle, Leinen), Seide, Wolle, Regeneratcellulose (Viskose, Rayon), Cellulosederivate als auch synthetische Fasern wie z.B. Polyester, Polyacrylnitril, Polyamid- oder Polyolefinfasern oder Mischungen solcher Fasern gewebt oder ungewebt verwendet werden. Diese Fasern können zu saugfähigen Wattepads, Vliesstoffen oder zu Geweben oder Gewirken verarbeitet sein. Auch flexible und saugfähige polymere Schaumstoffe, z. B. Polyurethanschäume und Polyamidschäume sind geeignete Substrate. Das Substrat kann eine, zwei, drei sowie mehr als drei Lagen aufweisen, wobei die einzelnen Lagen aus gleichen oder unterschiedlichen Materialien bestehen können. Jede Substratschicht kann eine homogene oder eine inhomogene Struktur mit beispielsweise verschiedenen Zonen unterschiedlicher Dichte aufweisen.
Als saugfähig im Sinne der Erfindung sind solche Trägersubstrate anzusehen, die bei 20° C wenigstens 10 Gew.-%, bezogen auf das Trockengewicht, an Wasser adsorptiv bzw. kapillar binden können. Bevorzugt eignen sich aber solche Träger, die wenigstens 100 Gew.-% Wasser adsorptiv und kapillar binden können.
Die Ausrüstung der Trägersubstrate erfolgt in der Weise, daß man die saugfähigen, flexiblen Trägersubstrate, bevorzugt aus Textilfasern, Kollagen oder polymeren Schaumstoffen mit den erfindungsgemäßen Zusammensetzungen behandelt bzw. ausrüstet und gegebenenfalls trocknet. Dabei kann die Behandlung (Ausrüstung) der Trägersubstrate nach beliebigen Verfahren, z. B. durch Aufsprühen, Tauchen und Abquetschen, Durchtränken oder einfach durch Einspritzen der erfindungsgemäßen Zusammensetzung in die Trägersubstrate erfolgen.

Erfindungsgemäß bevorzugt ist weiterhin die Darreichungsform als Aerosol, wobei die kosmetische Zusammensetzung ein Treibmittel, ausgewählt aus Propan, Butan, Isobutan, Pentan, Isopentan, Dimethylether, Fluorkohlenwasserstoffen und Fluorchlorkohlenwasserstoffen sowie Mischungen hiervon enthält.

Die folgenden Beispiele sollen die Erfindung verdeutlichen, ohne sie hierauf zu beschränken.

### Beispielrezepturen

| **Wasserfreie tensidhaltige AT-Stifte (Angaben in Gewichtsteilen)** | | | | | | | | | |
|---|---|---|---|---|---|---|---|---|---|
| | **1.1** | **1.2** | **1.3** | **1.4** | **1.5** | **1.6** | **1.7** | **1.8** | **1.9** |
| Eutanol® G 16 | 10 | - | - | 15 | 10 | - | 10 | - | 10 |
| Cetiol® OE | - | 10 | 15 | - | - | - | - | - | - |
| Ucon Fluid® AP | 5 | 5 | 5 | 5 | 5 | 5 | 5 | 5 | 5 |
| Cutina® HR | 6 | 6 | 6 | 6 | 6 | 6 | 2 | 5 | 6 |
| Lorol® C 18 | 20 | 20 | 20 | - | 20 | 20 | - | - | 20 |
| Lanette® O | - | - | - | 20 | - | - | 10 | 12 | - |
| Eumulgin® B 3 | 3 | 3 | 3 | 3 | 3 | 3 | 3 | 3 | - |
| Cutina® E 24 PF | - | - | - | - | 5 | - | - | - | - |
| Aluminiumchlorohydrat | 20 | 20 | 20 | 20 | 20 | 20 | 20 | - | - |
| Talkum | 8 | 8 | 8 | 8 | 8 | 8 | 8 | 28 | 28 |
| Cibafast H | 0,1 | - | - | - | - | - | - | - | - |
| Lävulinsäure | - | 0,5 | - | - | - | - | - | - | - |
| Zinklactat | - | - | 0,1 | - | - | - | - | - | - |
| Zinkgluconat | - | - | - | 0,1 | - | - | - | - | - |
| α-Bisabolol | - | - | - | - | 0,05 | - | - | - | - |
| Coenzym Q10-Konzentrat 3%ig | - | - | - | - | - | 0,2 | - | - | - |
| Deolite | - | - | - | - | - | - | 1,0 | - | - |
| Palmarosaöl | - | - | - | - | - | - | - | 1,5 | - |
| Chondroitinsulfat 6%ig | - | - | - | - | - | - | - | - | 0,2 |
| Siliconöl DC® 245 | ad 100 | ad 100 | ad 100 | ad 100 | ad 100 | ad 100 | ad 100 | ad 100 | ad 100 |

| | **1.10** | **1.11** | **1.12** | **1.13** | **1.14** | **1.15** | **1.16** | **1.17** | **1.18** |
|---|---|---|---|---|---|---|---|---|---|
| Eutanol® G 16 | 10 | - | - | 15 | 10 | - | 10 | - | 10 |
| Cetiol® OE | - | 10 | 15 | - | - | - | - | - | - |
| Ucon Fluid® AP | 5 | 5 | 5 | 5 | 5 | 5 | 5 | 5 | 5 |
| Cutina® HR | 6 | 6 | 6 | 6 | 6 | 6 | 2 | 5 | 6 |
| Lorol® C 18 | 20 | 20 | 20 | - | 20 | 20 | - | - | 20 |
| Lanette® O | - | - | - | 20 | - | - | 10 | 12 | - |
| Eumulgin® B 3 | 3 | 3 | 3 | 3 | 3 | 3 | 3 | 3 | - |
| Cutina® E 24 PF | - | - | - | - | 5 | - | - | - | - |
| Aluminiumchlorohydrat | 20 | 20 | 20 | 20 | 20 | 20 | 20 | - | - |
| Talkum | 8 | 8 | 8 | 8 | 8 | 8 | 8 | 28 | 28 |
| Pygeum Extract | 0,5 | - | - | - | - | - | - | - | - |
| Sepivinol R | - | 0,5 | - | - | - | - | - | - | - |
| Willowherb Extrakt | - | - | 0,5 | - | - | - | - | - | - |
| Xyleine | - | - | - | 0,5 | - | - | - | - | - |
| Phytinsäure | - | - | - | - | 0,1 | - | - | - | - |
| Baysilone® CF -1301 | - | - | - | - | - | 0,5 | - | - | - |
| Uvinul® N539T | - | - | - | - | - | - | 0,3 | - | - |
| ID 166 | - | - | - | - | - | - | - | 0,2 | - |
| Oleanoline DPG | - | - | - | - | - | - | - | - | 0,5 |
| Siliconöl DC® 245 | ad 100 | ad 100 | ad 100 | ad 100 | ad 100 | ad 100 | ad 100 | ad 100 | ad 100 |

| **Sprühfähige, translucente Antitranspirant-Mikroemulsionen (Angaben in Gew.-%)** | | | | | | | | |
|---|---|---|---|---|---|---|---|---|
| | **2.1** | **2.2** | **2.3** | **2.4** | **2.5** | **2.6** | **2.7** | **2.8** |
| Plantaren® 1200 | 1,71 | 1,71 | - | 1,71 | 1,71 | - | 1,71 | 1,71 |
| Plantaren® 2000 | 1,14 | 1,39 | 2,40 | 1,14 | 1,39 | 2,40 | 1,14 | 1,39 |
| Glycerinmonooleat | 0,71 | 0,71 | - | 0,71 | 0,71 | - | 0,71 | 0,71 |
| Dioctylether | 4,00 | 4,00 | 0,09 | 4,00 | 4,00 | 0,09 | 4,00 | 4,00 |
| Octyldodecanol | 1,00 | 1,00 | 0,02 | 1,00 | 1,00 | 0,02 | 1,00 | 1,00 |
| Parfümöl | 1,00 | 1,00 | 1,00 | 1,00 | 1,00 | 1,00 | 1,00 | 1,00 |
| Aluminiumchlorohydrat | 8,00 | 5,00 | 5,00 - | | - | - | 8,00 | 5,00 |
| 1,2-Propylenglycol | 5,00 | 5,00 | - | 5,00 | 5,00 | - | 5,00 | 5,00 |
| Glycerin | - | - | 5,00 | - | - | 5,00 | - | - |
| Cutina® E24 | 1,0 | - | - | - | - | - | - | - |
| Dehymuls® PGPH | - | 0,6 | - | - | - | - | - | - |
| Dermsoft® GMCY | - | - | 0,01 | - | - | - | - | - |
| Aluminiumgluconat | - | - | - | 1,0 | - | - | - | - |
| Mangangluconat | - | - | - | - | 1,2 | - | - | - |
| Magnesiumglucoheptonat | - | - | - | - | - | 1,7 | - | - |
| Rosmarinextrakt-NC (Dr. Marcus) | 8% | - | - | - | - | - | 0,01 | - |
| Lameform® TGI | - | - | - | - | - | - | - | 1,0 |
| Wasser | ad 100 | ad 100 | ad 100 | ad 100 | ad 100 | ad 100 | ad 100 | ad 100 |

| **Seifenhaltige Stifte (Angaben in Gew.-%)** | | | | | | | | |
|---|---|---|---|---|---|---|---|---|
| | **3.1** | **3.2** | **3.3** | **3.4** | **3.5** | **3.6** | **3.7** | **3.8** |
| Ethanol | 22,5 | 22,5 | 22,5 | 22,5 | 22,5 | 22,5 | 22,5 | 22,5 |
| Cutina® FS 45 | 4,4 | 4,4 | 4,4 | 4,4 | 4,4 | 4,4 | 4,4 | 4,4 |
| 1,3 Butandiol | 31,7 | 31,7 | 31,7 | 31,7 | 31,7 | 31,7 | 31,7 | 31,7 |
| 1,2 Propylenglykol | 21,0 | 21,0 | 21,0 | 21,0 | 21,0 | 21,0 | 21,0 | 21,0 |
| Eutanol® G | 2,0 | 2,0 | 2,0 | 2,0 | 2,0 | 2,0 | 2,0 | 2,0 |
| Aethoxal® B | 3,0 | 3,0 | 3,0 | 3,0 | 3,0 | 3,0 | 3,0 | 3,0 |
| Cremophor® RH 455 | 0,05 | 0,05 | 0,05 | 0,05 | 0,05 | 0,05 | 0,05 | 0,05 |
| NaOH 45 %ig | 1,44 | 1,44 | 1,44 | 1,44 | 1,44 | 1,44 | 1,44 | 1,44 |
| Parfümöl | 2,0 | 2,0 | 2,0 | 2,0 | 2,0 | 2,0 | 2,0 | 2,0 |
| Tinogard TL | 0,3 | - | - | - | - | - | - | - |
| Aluminiumgluconat | - | 1,0 | - | - | - | - | - | - |
| Puramax ZN | - | - | 0,2 | - | - | - | - | - |
| Polyplant® ME | - | - | - | 0,7 | - | - | - | - |
| Grüner Tee Extrakt | - | - | - | - | 0,015 | - | - | - |
| Zinkgluconat | - | - | - | - | - | 0,05 | - | - |
| Proteol OAT | - | - | - | - | - | - | 0,02 | - |
| Zinkglycinat | - | - | - | - | - | - | - | 1,0 |
| Wasser dest. | ad 100 | ad 100 | ad 100 | ad 100 | ad 100 | ad 100 | ad 100 | ad 100 |

| **Deodorant im Pumpzerstäuber (Angaben in Gew.-%)** | | | | | | | |
|---|---|---|---|---|---|---|---|
| | **4.1** | **4.2** | **4.3** | **4.4** | **4.5** | **4.6** | **4.7** |
| Ethanol 96 %-ig, (DEP vergällt) | 55,0 | 55,0 | 55,0 | 55,0 | 55,0 | 55,0 | 55,0 |
| Triethylcitrat | 3,0 | 3,0 | 3,0 | 3,0 | 3,0 | 3,0 | 3,0 |
| Cremophor® RH 455 | 0,5 | 0,5 | 0,5 | 0,5 | 0,5 | 0,5 | 0,5 |
| Eucarol® AGE-EC | 1,0 | - | - | - | - | - | - |
| Eucarol® AGE-ET | - | 0,2 | - | - | - | - | - |
| APG-Monolaurate | - | - | 0,5 | - | - | - | - |
| Emuliance | - | - | - | 0,1 | - | - | - |
| Dermsoft GMCY | - | - | - | - | 1,3 | - | - |
| Flavonoid Complex SC | - | - | - | - | - | 0,5 | - |
| Xyliance | - | - | - | - | - | - | 1,0 |
| Parfümöl | 1,0 | 1,0 | 1,0 | 1,0 | 1,0 | 1,0 | 1,0 |
| Wasser | ad 100 | ad 100 | ad 100 | ad 100 | ad 100 | ad 100 | ad 100 |

| **Wasserfreies Deodorant-Spray (Angaben in Gew.-%)** | | | | |
|---|---|---|---|---|
| | **6.1** | **6.2** | **6.3** | **6.4** |
| 2-Octyldodecanol | 0,5 | 0,5 | 0,5 | 0,5 |
| Ethanol 99 %-ig, (DEP vergällt) | 39 | 39,45 | 39 | 39 |
| Tinogard TT | 0,5 | - | - | - |
| Centella Asiatica | - | 0,05 | - | - |
| Protectate HR | - | - | 0,5 | - |
| Palmarosaöl | - | - | - | 0,5 |
| n-Butan | 60 | 60 | 60 | 60 |

| **Antitranspirant Roll-on (Angaben in Gew.-%)** | | | | | |
|---|---|---|---|---|---|
| | **5.1** | **5.2** | **5.3** | **5.4** | **5.5** |
| Ethanol 96 %-ig,(DEP vergällt) | 30,0 | 30,0 | 30,0 | 30,0 | 30,0 |
| Mergital® CS 11 | 2,0 | 2,0 | 2,0 | 2,0 | 2,0 |
| Eumulgin® B 3 | 2,0 | 2,0 | 2,0 | 2,0 | 2,0 |
| Aluminiumchlorohydrat | 20,0 | 20,0 | 20,0 | 20,0 | 20,0 |
| Hydroxyethylcellulose | 0,5 | 0,5 | 0,5 | 0,5 | 0,5 |
| Protectate® HR | 0,01 | - | - | - | - |
| Protelan AGL 95 PV | - | 2,0 | - | - | - |
| Ederline AAPP1-H | - | - | 0,4 | - | - |
| Ederline AAPP1-L | - | - | - | 0,7 | - |
| Emblica | - | - | - | - | 0,015 |
| Parfümöl | 0,8 | 0,8 | 0,8 | 0,8 | 0,8 |
| Wasser | ad 100 | ad 100 | ad 100 | ad 100 | ad 100 |

| **Antitranspirant-Spray vom Suspensionstyp (Angaben in Gew.-%)** | | | |
|---|---|---|---|
| | **6.1** | **6.2** | **6.3** |
| DC-245 | 10,0 | 10,0 | 10,0 |
| Isopropylmyristat | 5,0 | 5,0 | 5,0 |
| Aluminiumchlorohydrat-Pulver | 5,0 | 5,0 | 5,0 |
| Aerosil® R 972 | 2,0 | 2,0 | 2,0 |
| Meeresalgenextrakt | 0,5 | - | - |
| Henna Extrakt | - | 0,1 | - |
| Oleanoline DPG | - | - | 0,5 |
| n-Butan | ad 100 | ad 100 | ad 100 |

### Antitranspirant-Tücher

Für die erfindungsgemäße Ausführungsform als Antitranspirant-Tuch wurde ein einlagiges Substrat aus 100 % Viskose mit einem Flächengewicht von 50 g/m² mit jeweils 75 g der Beispielemulsionen 2.1 bzw. 2.2 bzw. 2.3 pro Quadratmeter oder mit jeweils 75 g der Beispiellösungen 4.1 bzw. 4.2 beaufschlagt, in Tücher geeigneter Größe geschnitten und in Sachets verpackt.

| **Rohstoff** | **Beschreibung/INCl** | **Herstellerfirma** |
|---|---|---|
| Aethoxal® B | PPG-5-Laureth-5 | Cognis |
| APG-Monolaurate | | Cognis |
| Baysilone CF-1301 | Phenylpropylsiloxysilicate | GE Silicones |
| Centella Asiatica | | Alchem |
| Cetiol® OE | Dicaprylyl Ether | Cognis |
| Cibafast H | Sodium Benzotriazoyl Sulfonate, Buteth-3, Tributyl Citrate | CIBA |
| Coenzym 010-Konzentrat 3%ig | Coenzym Q10 | |
| Cremophor® RH 455 | PEG-40 Hydrogenated Castor Oil, Aktivsubstanz 90 % | BASF |
| Cutina E 24 | PEG-20 Glyceryl Stearate | Cognis |
| Cutina® FS 45 | Palmitic Acid, Stearic Acid, Aktivsubstanz mind. 92 % | Cognis |
| Cutina® HR | Hydrogenated Castor Oil | Cognis |
| DC® 245 | Cyclopentasiloxane | Dow Corning |
| Dehymuls PGPH | Polyglyceryl-2 Dipolyhydroxystearate | Cognis |
| Deolite | 2,2-Dimethyl-3-phenyl-1-propanol (and) 1,2-Pentandiol | Dragoco |
| Dermsoft GMCY | Glyceryl Caprylate | Cognis |
| | | |
| Ederline AAPP1-H | PEG 40-Hydrogenated Castor Oil, PPG-2 Ceteareth-9, Apple Extract | Seporga |
| Ederline AAPP1-L | Hexyl Decanol, Apple Extract | Seporga |
| Emblica | Phyllanthus emblica | Merck KGaA |
| Emuliance | Cetearyl Wheat Bran Glycosides, Cetearyl alcohol | Soliance |
| Eucarol® AGE-EC | Disodium Cocopolyglucose Citrate | Cesalpinia Chemicals |
| Eucarol® AGE-ET | Sodium Alkylpolyglucose Tartrate | Cesalpinia Chemicals |
| Eumulgin® B 3 | Ceteareth-30 | Cognis |
| Eutanol® G 16 | Hexyldecyl Stearate | Cognis |
| Flavonoid Complex SC | Water, Butylene Glycol, Gingko Biloba Leaf Extract, Phenoxyethanol, Methyl Paraben, Ethyl Paraben, Propyl Paraben, Butyl Paraben, Isobutyl Paraben, ca. 2 % Flavonoid-Gehalt | Cosmetochem |
| Grüner Tee Extrakt (pulverförmig) | | Dragoco |
| ID 166 | Alkyl-2-hydroxydiphenylether-Derivat | CIBA |
| Lameform® TGI | Polyglyceryl-3 Diisostearate | Cognis |
| Lanette® O | Cetearyl Alcohol | Cognis |
| Lorol® C 18 | Stearyl Alcohol | Cognis |
| Mate (pulverförmig) | | Dragoco |
| Mergital® CS 11 | Ceteareth-11 | Cognis |
| Oleanoline DPG | Dipropylene glycol, Olea europaea (Olive) Leaf Extract | Cognis |
| Palmarosaöl | Cymbopogon Martini Oil | Robertet S.A. |
| Plantaren® 1200 | LAURYL GLUCOSIDE, ca. 50 % Aktivsubstanz | Cognis |
| Plantaren® 2000 | DECYL GLUCOSIDE, ca. 50 % Aktivsubstanz | Cognis |
| Polyplant® ME | Melissenextrakt | Polygon |
| Protectate HR | Riechstoffgemisch | Haarmann & Reimer |
| Protelan AGL 95 PV | Natriumlauroylglutamat-Pulver, ca. 3% Wasser, ca. 10% NaCl | Zschimmer & Schwarz GmbH & Co KG |
| Proteol OAT | Sodium Lauroyl Oat Amino Acids | Seppic |
| Puramax ZN | Zinc Lactate | |
| Pygeum Extract | Pygeum Extract | Alchem |
| Rosmarinextrakt 8% NC | Rosmarinextrakt wasserlöslich Pulver mit Standardmenge an 5% Rosmarinsäure, 3% Apigenin-7-glycosid | Dr. Marcus |
| Sepivinol R | Wine Extract | Seppic |
| Tinogard HS | Sodium Benzotriazoyl Butylphenol Sulfonate, | Ciba |
| Tinogard TL | Benzotriazolyl Dodezyl p-Cresol | Ciba |
| Tinogard TT | Tetradibutyl Pentaerithrityl Hydroxycinnamate | Ciba |
| Ucon Fluid® AP | PPG-14 Butyl Ether | Amerchol (Union Carbide) |
| Uvinul N539T | Octocrylene | BASF |
| Weidenröschen-Extrakt | Willowherb Extract | Dragoco |
| Xyleine | Xylitol and Citrus Medica Limonum (Lemon) Fruit Extract and Carica Papaya (Papaya) fruit Extract and Olea europaea (Olive) Leaf Extract | Vincience |
| Xyliance | Cetearyl Wheat Straw Glycosides and Cetearylalcohol | Soliance |

### Überprüfung der inhibitorischen Wirkung der Lipase-Inhibitoren (in vitro)

Die hemmende Wirkung der Enzym-Inhibitoren wurde *in vitro* an einem geeigneten Modellenzym nachgewiesen. Da eine hautrelevante Lipase nicht zur Verfügung stand, diente die Lipase (Fa. Sigma) aus *Pseudomonas species* als Modellenzym, um die Hemmwirkung der erfindungsgemäß verwendeten Inhibitoren zu testen. Als Referenz diente Hinokitiol.

### Durchführung:

Der Nachweis der Lipase-Enzymaktivität erfolgt analog zu den Angaben im *Lipase Color Liquid Autom* ―Datenblatt der Fa. Sentinel. Hierbei wird die Lipase-katalysierte Spaltung von 1,2-O-dilauryl-rac-glycero-3-glutarsäure-(6'-methylresorufin)-ester in Glutarsäure und Methylresorufin bestimmt. Die Freisetzung des Methylresorufin verursacht eine Purpurfärbung (λ = 580 nm), deren Bildung photometrisch verfolgt wird.

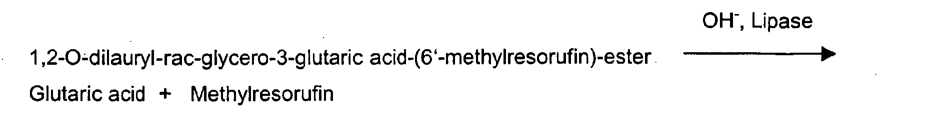

Der Assay wurde nach Herstellerangaben (37°C, Gesamtvolumen 1.21 ml) durchgeführt und enthielt: 37 mM Tris-Puffer (pH 8.4), 1,6 mM Desoxycholat, 6.6 mM Taurodesoxycholat, 0.9 mg/l Colipase, 1.5 mM Tartrat, 0.1 mM CaCl₂ und 0.24 mM 1,2-O-dilauryl-rac-glycero-3-glutaric acid-(6'-methylresorufin)-ester (Substrat). Die Reaktion wurde mit 0.005 U Lipase (10 µl) gestartet und nach einer Inkubation von einer Minute die Zunahme der Absorption bei 540 nm über 2 min verfolgt. Der lineare Anstieg der Absorption (A) pro Zeiteinheit (t) war hierbei ein Maß für die Aktivität des Enzyms (ΔA/Δt). Die Aktivität des Enzyms in Abwesenheit eines Inhibitors, (ΔA₁/Δt₁ ), wurde als Referenz gleich 100% gesetzt. Unter analogen Bedingungen wurden die Aktivitäten in Gegenwart eines Inhibitors (ΔA₂/Δt₂) bestimmt. Die Hemmwirkung des Inhibitors bzw. die Minderung der Enzymaktivität entspricht dann: 100% - (ΔA₂/Δt₂ )/( ΔA₁/Δt₁ )%.

### Hemmwirkung auf die Lipase-Aktiviät

| **Protelan AGL95PV (v/v*)** | **Hemmwirkung (Minderung der Lipase-Aktivität)** |
|---|---|
| 0% | 0 % |
| 0,01 % | 29 |
| 0,1 % | 56 |
| 1 % | 85 |

| | |
|---|---|
| *: volume per volume | |

## Patentansprüche

1. Verwendung von mindestens einer Lipase-inhibierenden Substanz, ausgewählt aus
- einbasigen C₂-C₈-Carbonsäuren mit 1 - 7 Hydroxygruppen und deren physiologisch verträglichen Salzen,
- aromatischen Carbonsäuren mit 6 - 24 Kohlenstoffatomen, 1 Carboxylgruppe, 1 - 2 Phenylresten, 1 - 6 Hydroxy- und/oder Cyanogruppen sowie deren physiologisch verträglichen Salzen,
- C₂-C₁₁-Aminosäuren, deren N-C₂-C₂₂-Acylderivaten sowie deren physiologisch verträglichen Salzen,
- den Tetraestern von Pentaerythrit mit C₂-C₄-Carbonsäuren, die einen substituierten Arylrest enthalten,
- gewünschtenfalls ethoxylierten Mono- und Diestern von Glycerin und Polyglycerinen mit gewünschtenfalls hydroxylierten C₈-C₂₂-Fettsäuren,
- den Ethern und Estern von gewünschtenfalls alkylierten Mono-, Oligo- und Polysacchariden,
- Pflanzenextrakten, ausgewählt aus den Extrakten von Centella asiatica, Apfelkernen, den Früchten (Beeren) von Phyllanthus emblica, Meeresalgen, Henna, den Blättern des Olivenbaumes (Olea europaea), der Rinde des Afrikanischen Zwetschgenbaumes (Pygeum africanum, Prunus africana), Weidenröschen (Epilobium angustifolium, Willowherb), Papayasaft, Zitronen und Palmarosaöl (Cymbopogon Martini Oil),
- Flavonoiden,
- Polyphenolen,
- Ubichinonen,
- 2-(2H-Benzotriazol-2-yl)-6-alkylphenolderivaten,
- α-Bisabolol,
- 2,2-Dimethyl-3-phenyl-1-propanol,
- Papain (Papaya-Petidase I, EC 3.4.22.2), Chymopapain (Papaya-Petidase II, EC 3.4.22.6), Bromelain (EC 3.4.22.32 und EC 3.4.22.33), Ficin (Ficain, EC 3.4.22.3) und Asclepain (EC 3.4.22.7),
- Phenylpropyldimethylsiloxysilicaten sowie
- Aluminiumchlorohydrat,
in einer kosmetischen Deodorant- oder Antitranspirant-Zusammensetzung zur Verringerung des durch die hydrolytische Zersetzung von natürlichen Fetten der Haut und der Kopfhaut verursachten Körpergeruchs.

2. Verwendung gemäß einem der vorhergehenden Ansprüche, **dadurch gekennzeichnet, dass** die physiologisch verträglichen Metallsalze ausgewählt sind aus den Ammo-nium-, Alkalimetall-, Magnesium-, Calcium-, Aluminium-, Zink- und Mangan-Salzen.

3. Verwendung gemäß Anspruch 1, **dadurch gekennzeichnet, dass** die einbasigen C₂-C₈-Hydroxycarbonsäuren mit 1 - 7 Hydroxygruppen ausgewählt sind aus Glycolsäure, Milchsäure, α-Hydroxybuttersäure, α-Hydroxyvaleriansäure, α-Hydroxycapronsäure, Gluconsäure, Galactonsäure, Mannonsäure, Fructonsäure, Arabinonsäure, Xylonsäure, Ribonsäure und Glucoheptonsäure sowie deren physiologisch verträglichen Salzen, insbesondere Zinklactat, Aluminiumlactat, Aluminiumgluconat, Zinkgluconat, Mangangluconat und Magnesiumglucoheptonat.

4. Verwendung gemäß einem der vorstehenden Ansprüche, **dadurch gekennzeichnet, dass** die aromatischen Carbonsäuren mit 6 - 24 Kohlenstoffatomen, 1 Carboxylgruppe, 1 - 2 Phenylresten, 1 - 6 Hydroxy- und/oder Cyanogruppen ausgewählt sind aus Mandelsäure, para-Hydroxymandelsäure, Rosmarinsäure, Ferulasäure, Kaffeesäure, Chlorogensäure, Salicylsäure, 2,3-Dihydroxybenzoesäure (Brenzcatechinsäure), 2,4-Dihydroxybenzoesäure (β-Resorcylsäure), 2,5-Dihydroxybenzoesäure (Gentisinsäure), 2,6-Dihydroxybenzoesäure (γ―Resorcylsäure), 3,4-Dihydroxybenzoesäure (Protocatechusäure), 3,5-Dihydroxybenzoesäure (α-Resorcylsäure), Gallussäure, 2-Cyano-3-phenylzimtsäure, den Methyl-, Ethyl- Isopropyl-, Propyl-, Butyl-, Hexyl-, Ethylhexyl-, Octyl-, Decyl-, Ethyloctyl-, Cetyl- und Stearylestern und den physiologisch verträglichen Salzen.

5. Verwendung gemäß Anspruch 1, **dadurch gekennzeichnet, dass** die C₂-C₁₁-Aminosäuren, deren N-C₂-C₂₂-Acylderivate sowie deren physiologisch verträgliche Metallsalze ausgewählt sind aus Aluminiumglycinat, Zinkglycinat, Zinkpyroglutamat, Natriumoctanoylglutamat, Natriumdecanoylglutamat, Natriumlauroylglutamat, Natriummyristoylglutamat, Natriumcetoylglutamat und Natriumstearoylglutamat und den Lauroyl-Derivaten von aus Getreidepflanzen gewonnenen Aminosäuren.

6. Verwendung gemäß Anspruch 1, **dadurch gekennzeichnet, dass** die Tetraester von Pentaerythrit mit C₂-C₄-Carbonsäuren, die einen substituierten Arylrest enthalten, ausgewählt sind aus Pentaerythrittetrakis[3(3,5-di-tert.-butyl-4-hydroxyphenyl)-propionat].

7. Verwendung gemäß Anspruch 1, **dadurch gekennzeichnet, dass** die gewünschtenfalls ethoxylierten Mono- und Diester von Glycerin und Polyglycerinen mit gewünschtenfalls hydroxylierten C₈-C₂₂-Fettsäuren ausgewählt sind aus PEG-20-glycerylstearat, Glycerylcaprylat, Polyglyceryl-2-dipolyhydroxystearat und Polyglyceryl-3-diisostearat.

8. Verwendung gemäß Anspruch 1, **dadurch gekennzeichnet, dass** die Ether und Ester von gewünschtenfalls alkylierten Mono-, Oligo- und Polysacchariden ausgewählt sind aus Dinatrium-Kokosalkylpolyglucosidcitrat (Disodium Cocopolyglucose Citrate), Natrium-Kokosalkylpolyglucosidtartrat (Sodium Cocopolyglucose Tartrate), den Veretherungsprodukten von C₈-C₂₂-Alkoholen mit Weizenstrohglycosiden, den Veretherungsprodukten von C₈-C₂₂-Alkoholen mit Weizenkleieglycosiden und Chondroitinsulfat.

9. Verwendung gemäß Anspruch 1, **dadurch gekennzeichnet, dass** die Flavonoide ausgewählt sind aus Naringin, α-Glucosylrutin, α-Glucosylmyricetin, α-Glucosylisoquercetin, α-Glucosylquercetin, Hesperidin, Neohesperidin, Rutin, Troxerutin, Monoxerutin, Diosmin, Eriodictin, Phloricin, Neohesperidindihydrochalkon und Apigenin-7-glucosid.

10. Verwendung gemäß Anspruch 1, **dadurch gekennzeichnet, dass** die Polyphenole ausgewählt sind aus Brenzcatechin, Resorcin, Hydrochinon, Phloroglucin, Pyrogallol, Hexahydroxybenzol, Anthocyanidinen, Flavonen, Gerbstoffen (Catechinen, Tanninen), Usninsäure, Acylpolyphenolen, den Derivaten der Gallussäure, den Derivaten der Digallussäure und der Digalloylgallussäure sowie den Polyphenolen aus Trauben und aus Rotwein.

11. Verwendung gemäß einem der vorstehenden Ansprüche, **dadurch gekennzeichnet, dass** die Ubichinone ausgewählt sind aus den Verbindungen der Formel (II), 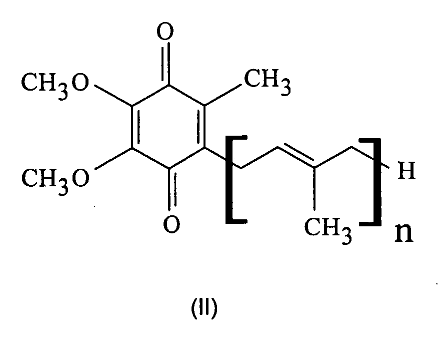 mit n = 6, 7, 8, 9 oder 10.

12. Verwendung gemäß Anspruch 1, **dadurch gekennzeichnet, dass** die 2-(2H-Benzotriazol-2-yl)-6-alkylphenolderivate ausgewählt sind aus Verbindungen der Strukturformel (III), 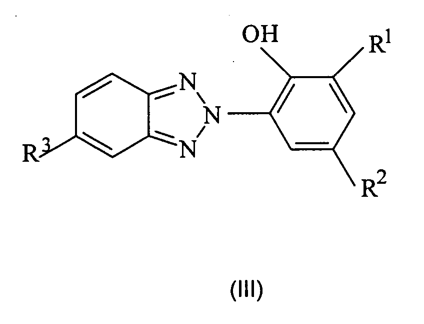 in der R¹ = eine lineare oder verzweigte C₁-C₁₂-Alkylgruppe,
R² = eine lineare oder verzweigte C₁-C₆-Alkylgruppe oder eine Sulfonsäuregruppe -SO₃X mit X = Wasserstoffatom oder ein einwertiges, physiologisch verträgliches Kation, und R³ = ein Atom H, F, Cl oder Br darstellen.

13. Verwendung gemäß Anspruch 12, **dadurch gekennzeichnet, dass** die 2-(2H-Benzotriazol-2-yl)-6-alkylphenolderivate ausgewählt sind aus 3-(2H-Benzotriazol-2-yl)-5-sec-butyl-4-phenolsulfonsäure-natrium, 2-(2H-Benzotriazol-2-yl)-6-dodecyl-4-methylphenol und 2-(5-Chloro-2H-benzotriazol-2-yl)-4-methyl-6-t-butylphenol.

14. Verfahren zur Verringerung von Körpergeruch mittels Inhibierung von Lipase auf der Haut, **dadurch gekennzeichnet, dass** eine kosmetische Deodorant- oder Antitranspirant-Zusammensetzung, enthaltend mindestens eine Lipase-inhibierende Substanz, ausgewählt aus
- einbasigen C₂-C₈-Carbonsäuren mit 1 - 7 Hydroxygruppen und deren physiologisch verträglichen Metallsalzen,
- aromatischen Carbonsäuren mit 6 - 24 Kohlenstoffatomen, 1 Carboxylgruppe, 1 - 2 Phenylresten, 1 - 6 Hydroxy- und/oder Cyanogruppen sowie deren physiologisch verträglichen Salzen,
- C₂-C₁₁-Aminosäuren, deren N-C₂-C₂₂-Acylderivaten sowie deren physiologisch verträglichen Metallsalzen,
- den Tetraestern von Pentaerythrit mit C₂-C₄-Carbonsäuren, die einen substituierten Arylrest enthalten,
- gewünschtenfalls ethoxylierten Mono- und Diestern von Glycerin und Polyglycerinen mit gewünschtenfalls hydroxylierten C₈-C₂₂-Fettsäuren,
- den Ethern und Estern von gewünschtenfalls alkylierten Mono-, Oligo- und Polysacchariden,
- Pflanzenextrakten, ausgewählt aus den Extrakten von Centella asiatica, Apfelkernen, den Früchten (Beeren) von Phyllanthus emblica, Meeresalgen, Henna, den Blättern des Olivenbaumes (Olea europaea), der Rinde des Afrikanischen Zwetschgenbaumes (Pygeum africanum, Prunus africana), Weidenröschen (Epilobium angustifolium, Willowherb), Papayasaft, Zitronen und Palmarosaöl (Cymbopogon Martini Oil),
- Flavonoiden,
- Polyphenolen,
- Ubichinonen,
- 2-(2H-Benzotriazol-2-yl)-6-alkylphenolderivaten,
- α-Bisabolol,
- 2,2-Dimethyl-3-phenyl-1-propanol,
- Papain (Papaya-Petidase I, EC 3.4.22.2), Chymopapain (Papaya-Petidase II, EC 3.4.22.6), Bromelain (EC 3.4.22.32 und EC 3.4.22.33), Ficin (Ficain, EC 3.4.22.3) und Asclepain (EC 3.4.22.7),
- Phenylpropyldimethylsiloxysilicaten sowie
- Aluminiumchlorohydrat,
auf die Haut, insbesondere auf die Haut der Achselhöhlen, aufgetragen wird.
